(19) Europäisches Patentamt
European Patent Office
Office européen des brevets

(11) **EP 4 445 859 A1**

(12) **EUROPEAN PATENT APPLICATION**

(43) Date of publication:
**16.10.2024 Bulletin 2024/42**

(21) Application number: **23305544.1**

(22) Date of filing: **12.04.2023**

(51) International Patent Classification (IPC):
**A61B 34/32** (2016.01)   **A61N 7/00** (2006.01)

(52) Cooperative Patent Classification (CPC):
**A61B 34/32; A61N 7/00;** A61N 2007/0021;
A61N 2007/0039; A61N 2007/0091;
A61N 2007/0095

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Commissariat à l'Energie Atomique et aux Energies Alternatives**
**75015 Paris (FR)**
• **Centre National de la Recherche Scientifique**
**75016 Paris (FR)**

• **Université de Strasbourg**
**67000 Strasbourg (FR)**

(72) Inventors:
• **SAOOD, Adnan**
**67000 Strasbourg (FR)**
• **VAPPOU, Jonathan**
**67100 Strasbourg (FR)**
• **NAGEOTTE, Florent**
**68920 Wettolsheim (FR)**
• **LARRAT, Benoit**
**75013 Paris (FR)**

(74) Representative: **IPAZ**
**Bâtiment Platon**
**Parc Les Algorithmes**
**91190 Saint-Aubin (FR)**

(54) **FOCUSED ULTRASOUND SONICATION WITH MECHANICAL AND ELECTRONIC STEERING ALONG A COMPUTED TRAJECTORY**

(57)   Computer-implemented method for controlling a device (1) intended to be used for opening a blood-brain barrier, comprising a step of calculating a trajectory and a step of controlling the device (1) to generate a focused ultrasound beam (12) producing a focal spot (13) and to move the focal spot (13) along said trajectory so as to cover a predetermined target volume.

**Fig. 1**

## Description

### Technical field

The present invention relates generally to computer-implemented methods for controlling devices generating focused ultrasound.

The invention is of particular interest for applications such as blood-brain barrier opening.

### Background

In the field of therapy for central nervous system diseases, techniques have been developed to temporarily disrupt the blood-brain barrier with focused ultrasound coupled with circulation of intravenously injected microbubbles.

While these techniques make it possible to open the blood-brain barrier in a reversible and non-invasive manner, they do generally not allow opening an extended volume of the blood-brain barrier, e.g. the volume of a tumor.

More generally, the known techniques are not suitable for some clinical applications.

### Description of the invention

To overcome the limitations of the prior art, the invention provides a computer-implemented method for controlling a device, in particular a medical device intended to be used for opening a blood-brain barrier, said device being configured to generate a focused ultrasound beam producing a focal spot and to allow electronic and/or mechanical steering of said focal spot, the method comprising the following steps:

- defining a trajectory of said focal spot based on one or more parameters selected from a list including a trajectory pattern, a travel speed of the focal spot and an acceleration of the focal spot,
- controlling the device to generate said focused ultrasound beam and to move the focal spot along said trajectory so as to cover a predetermined target volume.

According to the invention, it is thus proposed to calculate a trajectory of the focal spot based on a predetermined target volume, which can typically correspond to a volume inside which the blood-brain barrier needs to be open, defined by a physician.

This approach, of the inverse problem type, allows defining an appropriate or optimal opening trajectory, taking into account relevant parameters.

The inventors found that the proposed method makes it possible to increase the blood-brain barrier opening volume, as compared to conventional manual techniques.

In some embodiments, the step of controlling the device comprises interlacing mechanical steering and electronic steering of the focal spot.

Interlacing mechanical and electronic steering makes it possible to cover an extended volume in a reduced duration, in particular a duration that would be the same as, or close to, the one required to cover a surface forming a slice, or layer, of said volume.

Preferably, the step of controlling the device comprises generating the focused ultrasound beam with a series of ultrasound pulses.

Alternatively, the step of controlling the device may comprise generating a continuous focused ultrasound beam.

In some embodiments, the step of controlling the device comprises moving the focal spot between said ultrasound pulses.

One of the advantages of this particular type of interlacing is the reducing trajectory duration for a given target volume.

Of course, other types of interlacing mechanical and electronic steering can be implemented, for instance moving the focal spot during some of the ultrasound pulses, or more generally during ultrasound beam generation.

In some embodiments, said predetermined target volume is defined as a series of layers.

The layers can have a planar and/or curved geometry.

The layers can form continuous and/or discrete surfaces.

Preferably, said layers are spaced from each other along a reference direction.

The step of controlling the device may comprise generating the focused ultrasound beam along said reference direction.

In some embodiments, the step of defining the trajectory comprises calculating the trajectory of said focal spot for each of said layers.

Concerning the steering directions, the step of moving the focal spot may comprise electronic steering in said reference direction, also called first direction, and/or in a second direction and/or in a third direction, said first, second and third direction being perpendicular to each other, and/or in any corresponding rotation.

The step of moving the focal spot may comprise mechanical steering in the first direction and/or the second direction and/or the third direction, and/or in any corresponding rotation.

Various combinations of electronic and/or mechanical steering may be implemented, including the following non-limiting examples.

In some embodiments, the step of moving the focal spot comprises electronic steering in both first, second and third directions.

In some other embodiments, the step of moving the focal spot comprises mechanical steering in both first, second and third directions.

In still other embodiments, the step of moving the focal spot comprises electronic steering in said first direction and mechanical steering in said second and third directions.

In still other embodiments, the step of moving the focal spot comprises electronic steering in said first and third directions and mechanical steering in said second direction.

In still other embodiments, the step of moving the focal spot comprises electronic steering in said first direction and mechanical steering in said first, second and third directions.

In some embodiments, said trajectory pattern parameter includes a pattern-type parameter.

For example, the pattern-type parameter may be a spiral pattern or a pattern comprising multiple line sections parallel to each other.

Said trajectory pattern parameter includes a pattern-type parameter may optionally include a corresponding shape factor parameter.

For example, the shape factor parameter may be a distance between predetermined points of the pattern.

In some embodiments, the list from which said one or more parameters are selected includes a temporal evolution of a variable representative of a microbubble concentration and an acoustic pressure field.

In some embodiments, said travel speed and/or acceleration of the focal spot is calculated as a function of said temporal evolution of a variable representative of a microbubble concentration and/or of said acoustic pressure field.

In some embodiments, the step of defining the trajectory comprises simulating several trajectories and selecting one of these simulated trajectories.

Selecting one of the simulated trajectories is preferably made on the basis of one or more criteria such as a duration to complete the trajectory and/or a percentage of coverage of the target volume and/or a factor of homogeneity of exposure of the target volume.

According to another aspect, the invention provides a device having:

- a transducer configured to generate a focused ultrasound beam producing a focal spot,
- electronic and/or mechanical steering means configured to move said focal spot,
  and
- means adapted to execute the steps of a method as defined above.

In some embodiments, the device comprises a robotic arm bearing said transducer and forming said mechanical steering means.

According to yet another aspect, the invention provides a computer program comprising instructions to cause a device as defined above to execute the steps of a method as defined above.

According to yet another aspect, the invention provides a computer-readable storage medium having stored thereon a computer program as defined above.

The present invention, and all its aspects, embodiments and advantages associated therewith will become more readily apparent in view of the detailed description of particular embodiments provided below, including the accompanying drawings.

## Brief description of the drawings

In the following, non-limiting embodiments of the invention are described with reference to the accompanying drawings in which:

- Figure 1 is a schematic representation of a device according to the invention;
- Figure 2 is a schematic representation of a method according to the invention;
- Figure 3 is a schematic representation of a target volume defined by multiple layers;
- Figure 4 is a schematic representation of a layer of said target volume, and of a trajectory of a focal spot associated with this layer;
- Figure 5 is a schematic representation of a sonication scheme according to the invention.

## Detailed description of embodiments

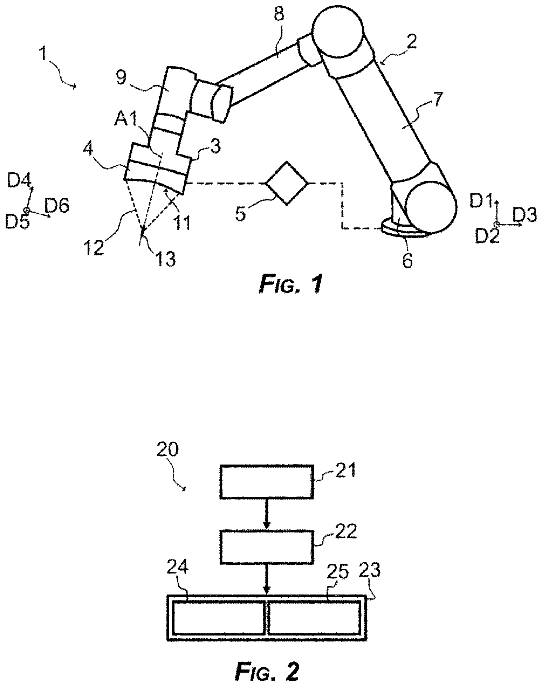

Figure 1 illustrates schematically a device 1 according to a non-limiting embodiment of the invention.

In this example, the device 1 is intended to be used for opening a blood-brain barrier.

The device 1 of figure 1 comprises a robotic arm 2, a holder 3, a transducer 4, and computer control means 5.

In this embodiment, the arm 2 comprises parts 6-9 connected to each other by pivotal links defining six degrees of freedom. More specifically, part 6, which forms a base of the arm 2, is connected to part 7 by two pivotal links. Parts 7 and 8 are connected to each other by one pivotal link. Part 8, which forms a terminal element of the arm 2, is connected to element 9 by three pivotal links. The transducer 4 is connected to the terminal element 8 of the arm 2 through the holder 3.

Figure 1 provides a first orthogonal spatial system formed by directions D1, D2 and D3, and a second orthogonal spatial system formed by directions D4, D5 and D6. The first system is associated with said base 6 of the arm 2 and the second system is associated with the transducer 4.

In this example, the transducer 4 is a conventional medical transducer configured to generate ultrasound pulses by means of an array of piezoelectric elements, whose phase can be modified individually, and which together define a concave active surface 11.

As known per se, such a transducer 4 allows to generate a focused ultrasound beam 12 so as to produce a focal spot 13, which typically has an ellipsoid shape of

a few mm$^3$. The position of the focal spot with respect to the active surface 11 can be modified by controlling the phase of the piezoelectric elements.

In this example, said piezoelectric elements are concentrically distributed about an axis A1 of the transducer 4, parallel to D4, allowing a modification of the focal spot position only in terms of focal distance, namely the distance between the active surface 11 and the focal spot 13 according to the transducer axis A1.

In the embodiment of figure 1, the arm 2 can be controlled to move the transducer 4 in any of the directions D1, D2 and D3, or in any combination of these directions and corresponding rotations, thanks to its degrees of freedom. The arm 2 therefore constitutes a mechanical steering means, in the sense that a mechanical displacement of its parts 7, 8 and 9 relative to part 6 allows to modify the position of the focal spot 13 according to D1 and/or D2 and/or D3.

In addition, the transducer 4 constitutes an electronic steering means, in the sense that a phase control of its piezoelectric elements allows to modify the position of the focal spot 13, in this example along the transducer axis A1 and then direction D4.

With reference to figure 1, said computer control means 5 is configured to control both the arm 2 and the transducer 4 in order to move the focal spot 13 mechanically and/or electronically.

The means 5 includes a computer-readable storage medium having stored thereon a computer program to control the arm 2 and the transducer 4 as described below or according to any other implemented method of the invention.

With reference to figure 2, the invention relates to a computer-implemented method 20 for controlling the device 1 of figure 1, or any other device suitable for implementing such a method.

In this example, the method 20 includes a step 21 of determining a target volume, a step 22 of defining a trajectory of the focal spot 13, and a step 23 of controlling the device 1, the latter being decomposed into a step 24 of generating said focused ultrasound beam 12 with the transducer 4 and a step 25 of moving the focal spot 13 along the trajectory defined in step 22, in order to cover said target volume determined in step 21.

Determining the target volume

In the example of blood-brain barrier opening application, the target volume can be beforehand defined by medical practitioners, typically using medical imaging.

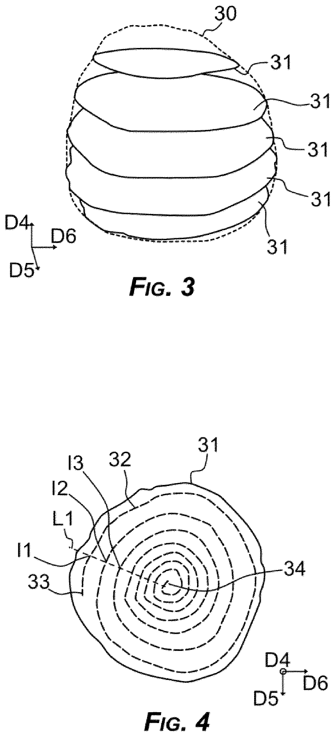

With reference to figure 3, the target volume 30 could have a relatively complex geometry, which can be approximated by an irregular polyhedral structure.

In this example, the computer program of the invention is an algorithm that defines the target volume 30 as a series of layers 31.

In the illustrative example of figure 3, the target volume 30 is here defined using five planar layers 31 spaced from each other along a reference direction, which corresponds in that case to the direction D4. Each of said layers 31 thus extends parallel to directions D5 and D6.

The algorithm determines how many layers 31 are needed to cover the target volume 30. This can be done using a predetermined distance between each pair of adjacent layers 31.

Defining the trajectory

In this example, trajectory solutions of the focal spot 13 are calculated for each of the layers 31. Figure 4 illustrates an example of trajectory 32 for one of the layer 31.

Trajectory solutions can be described in terms of trajectory pattern and of travel speed and/or acceleration of the focal spot 13.

One or multiple values for these parameters are typically stored and selected by the algorithm.

Concerning for example the parameter of speed or acceleration of the focal spot 13, the travel speed can be constant over time, corresponding to a zero acceleration of the focal spot 13, or can decrease according to a temporal function, for example in a constant or exponential way over time.

In the example of blood-brain barrier opening application, travel speed and acceleration of the focal spot 13 is preferably calculated as a function of a temporal evolution of a variable representative of a microbubble concentration. Actually, it is known in the art that the concentration of microbubbles decreases exponentially over time after their injection. The speed of the focal spot 13 can thus be correspondingly decreased over time to allow for longer sonication periods over tissue with lower microbubble concentration, ensuring compensation for said decreasing microbubble concentration.

With reference to the trajectory 32 of figure 4, the travel speed of the focal spot 13 is then decreased over time from the starting point 33 to the end point 34 of the trajectory 32.

In this example of application, travel speed and acceleration of the focal spot 13 can also be calculated as a function of an acoustic pressure field that can be estimated using known techniques.

Concerning the trajectory pattern parameter, it is in this example described in terms of a pattern-type and a corresponding shape factor.

In the example of figure 4, the pattern-type of the trajectory 32 is a spiral shape, i.e. a curve winding around a point - e.g. through which the transducer axis A1 passes - of which it is progressively getting closer, thus forming loops extending radially to each other.

The distance between said loops, in other words between predetermined parts or points I1, I2, I3... that can be formed by intersections of the trajectory 32 with a straight line L1 parallel to directions D5 and D6 and extending radially, may define a shape factor of the trajectory pattern. In the example of figure 4, the distance between two adjacent points l1, l2, l3... globally decreases from outside to inside the pattern, i.e. from the starting point 33 to the end point 34 of the trajectory 32.

In this example of a spiral pattern, the distance between loops of the spiral globally decreases with sonication progression, which may lead to an overlap of sonicated areas that takes into account the decrease over time of the microbubble concentration.

In other words, both the travel speed / acceleration parameter and the trajectory pattern parameter can be calculated as a function of the microbubble concentration evolution, whose decrease over time requires longer sonication to achieve in this example blood-brain barrier opening.

Therefore, the algorithm may first simulate trajectory solutions using several parameters, in this example travel speed / acceleration of the focal spot 13, trajectory pattern, temporal evolution of microbubble concentration and acoustic pressure field. The algorithm may then compare these simulated trajectory solutions to identify the trajectory having for example the best balance between overlap and sonication speed.

In this example, the algorithm calculates trajectory solutions for each of the layers 31, based on the condition of displacement of the focal spot 13 from one to another layer 31 between ultrasound pulses, the sonication being in this example pulse-width modulated with a duty cycle ranging for example from 1% to 10%.

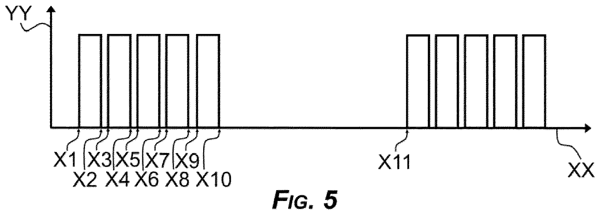

Such a sonication scheme, illustrated in figure 5, increases efficiency and ensures focused ultrasound energy deposition for cavitation in multiple depths during the same pulse-width modulated period.

Figure 5 illustrates a sonication scheme in a chart defining time in abscissa XX and focused ultrasound power in ordinate YY. The duration between time X1 and time X11 corresponds to a pulse-width modulated period. The duration between X1 and X2, respectively between X3 and X4, X5 and X6, X7 and X8, and X9 and X10, corresponds to a period during which a first, respectively a second, a third, a fourth, and a fifth, of said layers 31 are sonicated. The focal spot 13 is displaced from one to another layer 31 between ultrasound pulses, i.e. between X2 and X3, X4 and X5, X6 and X7, X8 and X9, and X10 and X11.

With reference to figures 3 and 4, the resultant trajectory is therefore a convolution of displacement of the focal spot 13 in directions D5 and/or D6 during ultrasound pulses, and displacement of the focal spot 13 in direction D4 between ultrasound pulses.

Simulation and optimization

The three-dimensional trajectory of the focal spot 13 can be written in a parametric form, in which elements of a vector $\Lambda$ are formed by parameters such as the travel speed of the focal spot 13 in a given layer 31, the shape factor - e.g. spiral width decay - and the initial value of the shape factor, and the distance between layers 31. Such a parametric form allows for designing an optimization scheme that iteratively modifies the parameters of the vector $\Lambda$.

In this example, a simulator is implemented to test each candidate trajectory. The simulator is preferably encapsulated inside a loss function to feedback into an optimization loop. The loss function can be a combination of total sonication time, coverage, and homogeneity, as defined by the following equation:

$$L_G(\Lambda) = Q\big(f(T), g(C), h(H)\big)$$

with $G$ a physical configuration (e.g. transducer and microbubble setup), $T$ a sonication duration, $C$ a coverage proportion defined as a floating point number $\in [0,1]$ equal to the area where sonication is larger than a predefined threshold and divided by the target volume, $H$ a homogeneity descriptor corresponding to a standard deviation of $I_{R_1}$ inside the target volume (see below), and where the functions $f$, $g$ and $h$ are designed to guide the optimizer toward a good point for the different targets of the optimization. In this example, these functions are simple linear functions with different weight values. $T$ can be calculated as the integration $\int_0^B dx/v(t)$, where $B$ is the trajectory length. $Q$ is a relational function that combines functions $f$, $g$, $h$ to create the result $L_G$. For example, and not limited to, $Q$ can result in an addition and/or multiplication relation between $f$, $g$, and $h$.

The following equation can be used to quantify the permeabilization of a point $X$ at a time $t$ after sonication for a duration $\tau_t$ under an acoustic pressure field $p(X, t)$ with a microbubble concentration c(t):

$$I_{R_1}(X) = M \int_t^{t+\tau_t} c(t) p(X, t) dt + A$$

The terms M and $A$ are additive and multiplicative terms that can be provided experimentally.

For the numerical simulation, the term $\Gamma$ can be formulated, $\Gamma$ being $I_{R_1}$ without the additive term $A$ and integrated temporally over the entire simulation session. When the acoustic pressure field is not covering point X, the term $p(X, t)$ is zero.

The quantity $\Gamma$ can be rewritten as a sum of exposure periods gathered only when $p(X, t) > 0$ over the entire sonication duration, i.e. when point X is inside the effective pressure field. As several sonications can deliver ultrasound cavitation energy to point X in an overlapped area, the quantity $\Gamma$ can be written in vectorized discrete form. The index $k$ below denotes the time samples that are combined for realizing a complete simulation iteration. Square and round brackets are for spatial vectorization and temporal discretization respectively.

$$\Gamma(X) = \sum_i \int_{t_i}^{t_i+\tau_i} c(t)p(X,t)dt$$

$$\Gamma_{k+1}[X] = \Gamma_k[X] + c(k\Delta t)p[X](k\Delta t)$$

The duration of sonication can be controlled by varying the velocity of the transducer 4. For a focal volume width in the movement direction of the transducer 4 of $W$ and velocity $v(t)$, the resultant sonication time is

$$\tau = \int_0^W dx/v(t)$$

, delivering cavitation energy calculable from pressure field profile $p(X, t)$.

After producing a simulation of a candidate trajectory described by the vector $\Lambda$, other trajectories can be calculated using a pattern-search algorithm, for instance the socalled Hooke-Jeeves pattern search algorithm.

The simulator output for a trajectory can be evaluated using appropriate metrics, e.g. operation duration, homogeneity of exposure, and coverage percentage. These metrics can be adjusted by the practitioner. A rating of the trajectory can be calculated, allowing the optimizer to adjust for a better point $\Lambda$ by modifying the trajectory parameters. The optimization iterations can stop after convergence.

An appropriate trajectory to cover the target volume 30 can be defined using the above-described principles, which are not limitative.

Generating and moving the focal spot

In the example of figures 1 and 2, the transducer 4 is controlled to generate the focused ultrasound beam 12 along the axis A1 with a series of ultrasound pulses, so as to produce the focal spot 13, and both the arm 2 and the transducer 4 are controlled to move the focal spot 13 along the trajectory defined in step 22 to cover the target volume 30.

With reference to figures 1, 3 and 4, and to the above description, displacement of the focal spot 13 within a given layer 31 is done by moving the arm 2 so as to displace the focal spot 13 in directions D5 and D6. In other words, sonicating a given layer 31 is accomplished by mechanical steering of the transducer 4. Displacement of the focal spot 13 from one to another layer 31 is done by electronic steering between ultrasound pulses.

In this example, the device 1 is thus controlled to interlace mechanical and electronic steering.

Many variations can be made to the above-described embodiments and principles. For example, the transducer 4 can comprise piezoelectric elements distributed both circumferentially and radially in relation to said transducer axis A1, allowing a modification of the focal spot 13 in both directions D4, D5 and D6. More generally, the focal spot 13 can be moved using any combination of mechanical and electronic steering, e.g. mechanical steering in only one of the directions D4, D5 and D6 and electronic steering in the two other direction.

As non-limiting alternatives, the transducer 4 can be controlled to generate a continuous focused ultrasound beam 12, and/or the target volume can be defined using non-planar layers, e.g. curved layers, and/or the pattern-type parameter may comprise a pattern having multiple line sections parallel to each other.

**Claims**

1. Computer-implemented method (20) for controlling a device (1), in particular a medical device intended to be used for opening a blood-brain barrier, said device (1) being configured to generate a focused ultrasound beam (12) producing a focal spot (13) and to allow electronic and/or mechanical steering of said focal spot (13), the method (20) comprising the following steps:

   - defining (22) a trajectory (32) of said focal spot (13) based on one or more parameters selected from a list including a trajectory pattern, a travel speed of the focal spot (13) and an acceleration of the focal spot (13),
   - controlling (23) the device (1) to generate (24) said focused ultrasound beam (12) and to move (25) the focal spot (13) along said trajectory (32) so as to cover a predetermined target volume (30).

2. Method (20) according to claim 1, wherein the step (23) of controlling the device (1) comprises interlacing mechanical steering and electronic steering of the focal spot (13).

3. Method (20) according to claims 1 or 2, wherein the step (23) of controlling the device (1) comprises generating (24) the focused ultrasound beam (12) with a series of ultrasound pulses.

4. Method (20) according to claim 3, wherein the step (23) of controlling the device (1) comprises moving (25) the focal spot (13) between said ultrasound pulses.

5. Method (20) according to anyone of claims 1 to 4, wherein said predetermined target volume (30) is defined as a series of layers (31).

6. Method (20) according to claim 5, wherein said layers (31) are spaced from each other along a reference direction (D4), the step (23) of controlling the device (1) preferably comprises generating (24) the focused ultrasound beam (12) along said reference direction (D4).

7. Method (20) according to claim 5 or 6, wherein the step (22) of defining the trajectory (32) comprises calculating the trajectory of said focal spot (13) for each of said layers (31).

8. Method (20) according to anyone of claims 1 to 7, wherein said trajectory pattern parameter includes:

   - a pattern-type parameter, for example a spiral pattern or a pattern comprising multiple line sections parallel to each other, and optionally
   - a corresponding shape factor parameter, for example a distance between predetermined points of the pattern.

9. Method (20) according to anyone of claims 1 to 8, wherein the list from which said one or more parameters are selected includes a temporal evolution of a variable representative of a microbubble concentration and an acoustic pressure field.

10. Method (20) according to claim 9, wherein said travel speed and/or acceleration of the focal spot (13) is calculated as a function of said temporal evolution of a variable representative of a microbubble concentration and/or of said acoustic pressure field.

11. Method (20) according to anyone of claims 1 to 10, wherein the step (22) of defining the trajectory (32) comprises simulating several trajectories and selecting one of these simulated trajectories on the basis of one or more criteria such as a duration to complete the trajectory (32) and/or a percentage of coverage of the target volume (30) and/or a factor of homogeneity of exposure of the target volume (30).

12. Device (1) having:

   - a transducer (4) configured to generate (24) a focused ultrasound beam (12) producing a focal spot (13),
   - electronic and/or mechanical steering means (2, 4) configured to move (25) said focal spot (13), and
   - means (5) adapted to execute the steps of a method (20) according to anyone of claims 1 to 11.

13. Device (1) according to claim 12, comprising a robotic arm (2) bearing said transducer (4) and forming said mechanical steering means.

14. Computer program comprising instructions to cause a device (1) according to claim 12 or 13 to execute the steps of a method (20) according to anyone of claims 1 to 11.

15. Computer-readable storage medium having stored thereon a computer program according to claim 14.

FIG. 1

FIG. 2

**FIG. 3**

**FIG. 4**

*FIG. 5*

| | Europäisches<br>Patentamt<br>European<br>Patent Office<br>Office européen<br>des brevets | | **EUROPEAN SEARCH REPORT** | | Application Number<br><br>**EP 23 30 5544** |
|---|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/118632 A1 (INSIGHTEC LTD [IL]; LEVY YOAV [IL] ET AL.) 7 August 2014 (2014-08-07) * paragraphs [0011], [0014], [0025], [0030], [0031]; figures 1,3 * | 1-9, 11-15 | INV.<br>A61B34/32<br>A61N7/00 |
| X | US 2013/018285 A1 (PARK JUN-HO [KR] ET AL) 17 January 2013 (2013-01-17) * figures 1,3A-C,7 * | 1-8, 12-15 | |
| X | WO 2022/188800 A1 (UNIV HONG KONG [CN]) 15 September 2022 (2022-09-15) * claim 1; figures 1Aa,2A * | 1-8, 12-15 | |
| X | US 2019/126317 A1 (PERNOT MATHIEU [FR] ET AL) 2 May 2019 (2019-05-02) * paragraphs [0031], [0038]; figures 1,3 * | 1-9, 12-15 | |
| X | US 2021/196295 A1 (GOUDOT GUILLAUME [FR] ET AL) 1 July 2021 (2021-07-01) * claim 1; figure 1 * | 1-8, 12-15 | TECHNICAL FIELDS SEARCHED (IPC) |
| X | CN 104 815 399 A (UNIV XI AN JIAOTONG) 5 August 2015 (2015-08-05) * claims 1,8; figures 1,2,3 * | 1-8, 12-15 | A61B<br>A61N |
| X | CN 101 058 005 A (UNIV HUAZHONG SCIENCE TECH [CN]) 24 October 2007 (2007-10-24) * the whole document * | 1-10, 12-15 | |

The present search report has been drawn up for all claims

| Place of search | Date of completion of the search | Examiner |
|---|---|---|
| The Hague | 12 September 2023 | Seon, Jean-Antoine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5544

12-09-2023

| Patent document cited in search report | | | Publication date | Patent family member(s) | | | Publication date |
|---|---|---|---|---|---|---|---|
| WO 2014118632 | A1 | | 07-08-2014 | CN | 105682739 | A | 15-06-2016 |
| | | | | EP | 2950737 | A1 | 09-12-2015 |
| | | | | EP | 4179995 | A2 | 17-05-2023 |
| | | | | JP | 6403689 | B2 | 10-10-2018 |
| | | | | JP | 2016509506 | A | 31-03-2016 |
| | | | | US | 2015359603 | A1 | 17-12-2015 |
| | | | | WO | 2014118632 | A1 | 07-08-2014 |
| US 2013018285 | A1 | | 17-01-2013 | CN | 102872542 | A | 16-01-2013 |
| | | | | EP | 2545963 | A1 | 16-01-2013 |
| | | | | JP | 2013022450 | A | 04-02-2013 |
| | | | | KR | 20130009138 | A | 23-01-2013 |
| | | | | US | 2013018285 | A1 | 17-01-2013 |
| WO 2022188800 | A1 | | 15-09-2022 | NONE | | | |
| US 2019126317 | A1 | | 02-05-2019 | CA | 3021814 | A1 | 26-10-2017 |
| | | | | CN | 109416907 | A | 01-03-2019 |
| | | | | EP | 3236467 | A1 | 25-10-2017 |
| | | | | EP | 3446306 | A1 | 27-02-2019 |
| | | | | IL | 262354 | A | 29-11-2018 |
| | | | | JP | 6980696 | B2 | 15-12-2021 |
| | | | | JP | 2019514485 | A | 06-06-2019 |
| | | | | KR | 20190004701 | A | 14-01-2019 |
| | | | | US | 2019126317 | A1 | 02-05-2019 |
| | | | | WO | 2017182655 | A1 | 26-10-2017 |
| US 2021196295 | A1 | | 01-07-2021 | CA | 3079920 | A1 | 02-05-2019 |
| | | | | CN | 111278506 | A | 12-06-2020 |
| | | | | EP | 3700629 | A1 | 02-09-2020 |
| | | | | FR | 3072577 | A1 | 26-04-2019 |
| | | | | US | 2021196295 | A1 | 01-07-2021 |
| | | | | WO | 2019081329 | A1 | 02-05-2019 |
| CN 104815399 | A | | 05-08-2015 | NONE | | | |
| CN 101058005 | A | | 24-10-2007 | NONE | | | |

EPO FORM P0459

For more details about this annex : see Official Journal of the European Patent Office, No. 12/82